# EUROPÄISCHE PATENTSCHRIFT

(11) **EP 0 645 355 B1**
(45) Veröffentlichungstag und Bekanntmachung des Hinweises auf die Patenterteilung: **22.03.2000**
(21) Anmeldenummer: 94114855.3
(22) Anmeldetag: 21.09.1994
(51) Int. Cl.: C07B 45/06, C07C 319/14, C07C 323/03, C07C 323/09, C07C 323/36, C07D 213/70, C07D 277/74

(54) **Verfahren zur Herstellung von Trifluorethylschwefelverbindungen aus Thiolaten und 1-Chlor-2,2,2-trifluorethan**
Method for the preparation of trifluoroethyl sulphur compounds from thiolates and 1-chloro-2,2,2-trifluoroethane
Procédé de préparation de composés trifluorosoufrés au départ de thiolates et de 1-chloro-2,2,2-trifluoroéthane

(30) Priorität: 29.09.1993 DE 4333058
(43) Veröffentlichungstag der Anmeldung: 29.03.1995
(73) Patentinhaber: SOLVAY (Société Anonyme), 1050 Bruxelles (BE)
(72) Erfinder: Appel, Wolfgang, Dr., D-65779 Kelkheim (DE); Ebmeyer, Frank, Dr., D-65795 Hattersheim (DE); Metzenthin, Tobias, Dr., D-65929 Frankfurt (DE); Siegemund, Günter, Dr., D-65719 Hofheim (DE)
(74) Vertreter: Jacques, Philippe

(56) Entgegenhaltungen:
- GB-A- 2 251 618
- BULLETIN OF THE CHEMICAL SOCIETY OF JAPAN, Bd.50, Nr.11, November 1977 Tokyo, JP Seiten 3069 - 3070 T. NAKAI, ET AL.: 'A convenient preparation of arylthioynamines'
- Tetrahedron, Vol. 48, No. 28, pp. 5823-5830, 'On the reactivity of CF3H3-nC2X (n=0,1,2 or 3 and X = H or halogen atom)', BODOR ET AL

## Beschreibung

T. Nakai et al, Bulletin of the Chemical Society of Japan, (1977), Bd. 50, Nr. 11, Seiten 3069-3070 offenbart die Herstellung von 2,2,2-Trifluoroethylthiobenzol, 4-Methyl-(2'2'2'-trifluoroethylthio)benzol und 2,2,2-trifluoroethylthioethan. Als Ausgangsprodukt wird Trifluoroethyl p-toluolsulfonat verwendet und als Lösungmittel DMF. Trifluoroethyl p-toluolsulfonat is jedoch nicht technisch leicht verfügbar.

Die bekanntermaßen sehr nucleophilen Thiolate ließen sich bisher mit dem technisch leicht verfügbaren 1-Chlor-2,2,2-trifluorethan (im folgenden kurz als Chlortrifluorethan oder R133a bezeichnet) nur in zwei Ausnahmefällen alkylieren. So wurde R133a mit einer wässrigen Lösung von Benzylmercaptan zum Benzyltrifluorethylthioether umgesetzt (GB-A-2,251,618, Beispiel 3). Allerdings wird diese Reaktion unter Druck in einem Bombenrohr durchgeführt und erfordert den Einsatz eines Phasentransferkatalysators. In einer anderen Veröffentlichung wurde nur die Umsetzung mit einem intermediär erzeugten Biphenyl, nämlich 2-(2'-Aminophenyl)thiophenol erwähnt (H. Shimizu et al ., J. Chem. Soc. Perkin Trans. 1, 1991, 1733-1747; Tabelle 1, Verbindung 3 v). Die Versuchsbeschreibung auf Seite 1741 erläutert jedoch die Alkylierung der Schwefelfunktion nur am Beispiel des reaktiven Methyliodids und berücksichtig nicht die bekannte Reaktionsträgheit von R133a. Allgemein wurde nämlich erwartet, daß Verbindungen vom Typ CF₃CH₂X sich als Alkylierungsmittel extrem unreaktiv verhalten (N. Bodor et al., Tetrahedron 48 [1992] 5823-5830 und T. Umemoto, Y. Gotoh, J. Fluorine Chem. 31 [1986] 231-236).

Es wurde nun überraschend gefunden, daß Chlortrifluorethan in einem polaren und aprotischen Lösungsmittel dennoch mit aliphatischen, aromatischen und heterocyclischen Thiolaten in guten Ausbeuten umgesetzt werden kann. Ein Katalysator ist dabei nicht notwendig. Außerdem kann die Umsetzung bereits bei einem geringen Druck bis 5 bar (einfache geschlossene Apparatur) oder gar völlig drucklos (Atmosphärendruck) durchgeführt werden.

Gegenstand der vorliegenden Erfindung ist somit ein Verfahren zur Herstellung von Trifluorethylschwefelverbindungen aus Thiolaten und 1-Chlor-2,2,2-trifluorethan, dadurch gekennzeichnet, daß man 1-Chlor-2,2,2-trifluorethan in einem polaren aprotischen Lösungsmittel auf Salze von Thiolen der Formel RSH einwirken läßt, wobei R ein unsubstituierter oder substituierter Rest mit folgender Bedeutung ist:
C₁-C₂₀-Alkyl
   oder
C₃-C₁₂-Cycloalkyl
   oder
ein einkerniger, kondensierter zweikerniger oder kondensierter dreikerniger Arylrest,
   oder
ein gesättigter oder ungesättigter ein- oder zweikerniger heterocyclischer Rest, der insgesamt ein oder zwei Heteroatome enthält, die unabhängig voneinander N, S oder O sein können.

Vorzugsweise wird die Reaktion unter Atmosphärendruck (offene Apparatur) oder unter einem Druck bis 5 bar (einfache geschlossene Apparatur) durchgeführt. Auch höherer Druck, z.B. 5 bis 10 bar, ist ohne weiteres möglich, aber nicht nötig.

Die hergestellten Trifluorethylschwefelverbindungen haben die Formel RSCH₂CF₃.

Im allgemeinen wird bei Temperaturen von 0 bis 100°C gearbeitet; eine höhere Reaktionstemperatur ist möglich, bietet aber keine Vorteile. Vorzugsweise wird die Umsetzung bei Temperaturen von 20 bis 70°C vorgenommen, insbesondere bei 40 bis 60°C. Das Chlortrifluorethan wird vorzugsweise im Überschuß eingesetzt, um die Reaktion zu beschleunigen und die Ausbeute zu erhöhen. Unverbrauchtes Chlortrifluorethan läßt sich nach der Umsetzung destillativ zurückgewinnen.

Geeignete polare aprotische Lösungsmittel sind z.B. N-Methylpyrrolidon, N-Methylformamid, Dimethylformamid, Dimethylacetamid, Sulfolan, Dimethylsulfoxid, Butyrolacton und Tetrahydrofuran. Von diesen Lösungsmitteln werden N-Methylformamid, Dimethylformamid, Dimethylacetamid, Dimethylsulfoxid und Butyrolacton vorzugsweise eingesetzt, insbesondere N-Methylpyrrolidon und Dimethylformamid.

Beim Einsatz von Thiolaten mit weiteren funktionellen Gruppen erfolgt die Umsetzung ausschließlich an der Schwefelfunktion, während beispielsweise Stickstoff- oder Carboxylatfunktionen, die ebenfalls nucleophil sind, nicht angegriffen werden.

Als Thiolate eignen sich alle üblichen Salze der genannten Thiole der Formel RSH.

Im allgemeinen hat R folgende Bedeutung:
C₁-C₂₀-Alkyl,
   unsubstituiert oder substituiert mit einem oder zwei Resten aus der Gruppe Amino, F, C₁-C₃-Alkylamino, C₁-C₃-Dialkylamino, Hydroxyl, Carboxyl, C₁-C₆-Alkoxy, C₃-C₁₂-Cycloalkyl, Pyridyl, Imidazolyl, Thiazolyl, Oxazolyl, Phenyl, wobei Phenyl unsubstituiert oder mit einem oder zwei Resten aus der Gruppe F, Cl, Br, Amino, C₁-C₃-Alkylamino, C₁-C₃-Dialkylamino, Hydroxyl, Carboxyl, C₁-C₃-Alkoxy, C₁-C₆-Alkyl substituiert ist;
   oder
C₃-C₁₂-Cycloalkyl,
   unsubstituiert oder substituiert mit einem oder zwei Resten aus der Gruppe C₁-C₆-Alkyl, F, Amino, C₁-C₃-Alkylamino, C₁-C₃-Dialkylamino, Hydroxyl, Carboxyl, C₁-C₆-Alkoxy, Pyridyl, Imidazolyl, Thiazolyl, Oxazolyl, Phenyl, wobei Phenyl unsubstituiert oder mit einem oder zwei Resten aus der Gruppe F, Cl, Br, Amino, C₁-C₃-Alkylamino, C₁-C₃-Dialkylamino, Hydroxyl, Carboxyl, C₁-C₃-Alkoxy, C₁-C₆-Alkyl substituiert ist;
   oder
ein einkerniger, kondensierter zweikerniger oder kondensierter dreikerniger Arylrest,
   unsubstituiert oder substituiert mit einem oder zwei Resten aus der Gruppe C₁-C₆-Alkyl, F, Cl, Br, Amino, C₁-C₃-Alkylamino, C₁-C₃-Dialkylamino, Hydroxyl, Carboxyl, C₁-C₆-Alkoxy;
   oder
ein gesättigter oder ungesättigter, ein- oder zweikerniger heterocyclischer Rest, der insgesamt ein oder zwei Heteroatome enthält, die unabhängig voneinander N, S oder O sein können,
   wobei der Rest unsubstituiert oder substituiert ist mit einem oder zwei Resten aus der Gruppe C₁-C₆-Alkyl, F, Amino, C₁-C₃-Alkylamino, C₁-C₃-Dialkylamino, Hydroxyl, Carboxyl, C₁-C₆-Alkoxy, sowie, falls der heterocyclische Rest aromatisch ist, Cl und Br.

Dabei bedeutet "C₁-C₃-Dialkylamino" eine mit zwei Alkylresten substituierte Aminogruppe, wobei die beiden Alkylreste unabhängig voneinander 1 bis 3 C-Atome haben.

Vorzugsweise hat R folgende Bedeutung:
C₁-C₁₂-Alkyl,
   unsubstituiert oder substituiert mit einem oder zwei Resten aus der Gruppe F, Amino, C₁-C₃-Alkylamino, C₁-C₃-Dialkylamino, Hydroxyl, Carboxyl, C₁-C₃-Alkoxy, C₃-C₈-Cycloalkyl, Pyridyl, Imidazolyl, Thiazolyl, Oxazolyl, Phenyl, wobei Phenyl unsubstituiert oder mit einem oder zwei Resten aus der Gruppe F, Cl, Br, Amino, C₁-C₃-Alkylamino, C₁-C₃-Dialkylamino, Hydroxyl, Carboxyl, C₁-C₃-Alkoxy, C₁-C₄-Alkyl substituiert ist;
   oder
C₃-C₈-Cycloalkyl,
   unsubstituiert oder substituiert mit einem oder zwei Resten aus der Gruppe C₁-C₄-Alkyl, F, Amino, C₁-C₃-Alkylamino, C₁-C₃-Dialkylamino, Hydroxyl, Carboxyl, C₁-C₃-Alkoxy, Pyridyl, Imidazolyl, Thiazolyl, Oxazolyl, Phenyl, wobei Phenyl unsubstituiert oder mit einem oder zwei Resten aus der Gruppe F, Cl, Br, Amino, C₁-C₃-Alkylamino, C₁-C₃-Dialkylamino, Hydroxyl, Carboxyl, C₁-C₃-Alkoxy, C₁-C₄-Alkyl substituiert ist;
   oder
ein einkerniger, kondensierter zweikerniger oder kondensierter dreikerniger Arylrest,
   unsubstituiert oder substituiert mit einem oder zwei Resten aus der Gruppe C₁-C₄-Alkyl, F, Cl, Br, Amino, C₁-C₃-Alkylamino, C₁-C₃-Dialkylamino, Hydroxyl, Carboxyl, C₁-C₃-Alkoxy;
   oder
ein gesättigter oder ungesättigter, ein- oder zweikerniger heterocyclischer Rest, der insgesamt ein oder zwei Heteroatome enthält, die unabhängig voneinander N, S oder O sein können,
   wobei der Rest unsubstituiert oder substituiert ist mit einem oder zwei Resten aus der Gruppe C₁-C₄-Alkyl, F, Amino, C₁-C₃-Alkylamino, C₁-C₃-Dialkylamino, Hydroxyl, Carboxyl, C₁-C₃-Alkoxy, sowie, falls der heterocyclische Rest aromatisch ist, Cl und Br.

Insbesondere hat R folgende Bedeutung:
C₁-C₆-Alkyl,
   unsubstituiert oder substituiert mit einem oder zwei Resten aus der Gruppe F, Amino, C₁-C₃-Alkylamino, C₁-C₃-Dialkylamino, Hydroxyl, Carboxyl, C₁-C₃-Alkoxy, C₃-C₈-Cycloalkyl, Pyridyl, Imidazolyl, Thiazolyl, Oxazolyl, Phenyl, wobei Phenyl unsubstituiert oder mit einem oder zwei Resten aus der Gruppe F, Cl, Br, C₁-C₃-Alkyl, C₁-C₃-Alkoxy substituiert ist;
   oder
C₃-C₆-Cycloalkyl,
   unsubstituiert oder substituiert mit einem oder zwei Resten aus der Gruppe C₁-C₄-Alkyl, F, Amino, C₁-C₃-Alkylamino, C₁-C₃-Dialkylamino, Hydroxyl, Carboxyl, C₁-C₃-Alkoxy, Pyridyl, Imidazolyl, Thiazolyl, Oxazolyl, Phenyl;
   oder
Phenyl,
   unsubstituiert oder substituiert mit einem oder zwei Resten aus der Gruppe C₁-C₃-Alkyl, F, Cl, Br, Amino, C₁-C₃-Alkylamino, C₁-C₃-Dialkylamino, Hydroxyl, Carboxyl, C₁-C₃-Alkoxy;
   oder
ein ungesättigter ein- oder zweikerniger heterocyclischer Rest, der insgesamt ein oder zwei Heteroatome enthält, die unabhängig voneinander N, S oder O sein können,
   wobei der Rest unsubstituiert oder substituiert ist mit einem oder zwei Resten aus der Gruppe C₁-C₃-Alkyl, F, Amino, C₁-C₃-Alkylamino, C₁-C₃-Dialkylamino, Hydroxyl, Carboxyl, C₁-C₃-Alkoxy, sowie, falls der heterocyclische Rest aromatisch ist, Cl und Br.

Vor allem hat R folgende Bedeutung:
C₁-C₄-Alkyl;
   oder
C₃-C₆-Cycloalkyl;
   oder
Phenyl,
   unsubstituiert oder substituiert mit einem oder zwei Resten aus der Gruppe Methyl, F, Cl, Br, Amino, C₁-C₃-Alkylamino, C₁-C₃-Dialkylamino, Hydroxyl, Carboxyl, Methoxy;
   oder
Pyridyl, Imidazolyl, Benzimidazolyl, Thiazolyl, Benzthiazolyl, Oxazolyl oder Benzoxazolyl.

Die beim erfindungsgemäßen Verfahren einzusetzenden Thiolate können allgemein durch Einwirkung äquimolarer Mengen verschiedener ein- oder zweiwertiger organischer oder anorganischer Basen, wie z.B. Alkali-, Erdalkali-, Ammonium- oder Alkylammoniumhydroxid, Natriumhydrid, Calciumhydrid, Alkali- oder Erdalkalialkoholat, Ammoniak, primäre, sekundäre oder tertiäre Alkyl-, Aryl- oder Aralkylamine, Amidine oder Pyridin, auf das entsprechende Thiol erzeugt werden. Dabei verwendet man als Base vorzugsweise Natriumhydroxid, Kaliumhydroxid, Pyridin, Ammoniak, primäre bis tertiäre Alkyl-sowie Aralkylamine oder Natriumhydrid. Insbesondere verwendet man Natriumhydroxid, Kaliumhydroxid, Natriumhydrid oder Alkylamine. Dabei lassen sich sowohl unter wasserfreien Reaktionsbedingungen als auch beispielsweise unter Einsatz von wäßriger Alkalilauge gute Ergebnisse erzielen. Das Thiolat kann vor der Reaktion hergestellt oder in situ erzeugt werden. Als Kation des eingesetzten Thiolats kommt demgemäß beispielsweise in Frage: Na⁺, K⁺, Ca²⁺, NR¹R²R³H⁺ (R¹⁻³ = C₁-C₆-n-Alkyl, C₁-C₆-Cycloalkyl, Aryl, H), C₅H₅NH⁺ (Pyridinium), C₆H₁₂N₂H⁺ ( Kation des Amidins Diazabicyclononen, DBN); vorzugsweise Na⁺, K⁺, NR¹R²R³H⁺ (R¹⁻³ = C₁-C₆-n-Alkyl, C₁-C₆-Cycloalkyl, Aryl, H), C₅H₅NH⁺;
insbesondere Na⁺, K⁺, NR¹R²R³H⁺ (R¹⁻³ = C₁-C₆-n-Alkyl, Aryl, H), C₅H₅NH⁺.

Die den Thiolaten zugrunde liegenden Thiole lassen sich allgemein durch Erhitzen von Schwefelwasserstoff oder seinen Metallsalzen mit organischen Halogenverbindungen herstellen, wobei die Halogenfunktion gegen die Mercaptogruppierung ausgetauscht wird.

Je nach Kation des eingesetzten Thiolats (Gegenion) fällt bei der Reaktion mit dem Chlortrifluorethan (R133a) das entsprechende ein- oder zweiwertige Metall-, Ammonium- bzw. Alkylammoniumchlorid als einziges Nebenprodukt an, von dem die erwünschten Trifluorethylschwefelverbindungen leicht (z.B. durch Filtration) abgetrennt werden können. Ein Vorteil der erfindungsgemäßen Verfahrens ist daher, daß keine jod- oder bromhaltigen Salze entstehen, die schwer zu entsorgen wären.

Als Werkstoffe für das Reaktionsgefäß kommen alle hinreichend inerten Materialien wie z.B. unlegierter Stahl, Nickel, Hastelloy, sowie Glas infrage.

Die erfindungsgemäße Methode zur Herstellung von Trifluorethylschwefelverbindungen erweist sich als kostensparend, da sie ein drucklos oder bei niedrigem Druck und einstufig durchführbares Eintopfverfahren darstellt. Die hergestellten Verbindungen sind als Zwischen- und Endprodukte von Pflanzenschutz- und Pharmawirkstoffen einsetzbar.

Eine geeignete Vorgehensweise zur Herstellung der Trifluorethylschwefelverbindungen besteht darin, daß man das gewählte Thiol in einem geeigneten Lösungmittel unter Inertgas vorlegt und mit Alkalimetallhydroxid in das Thiolat verwandelt. Anschließend leitet man bei Raumtemperatur die erforderliche Menge Chlortrifluorethan aus einem Vorratsbehälter in die Lösung ein und heizt dann auf die Reaktionstemperatur. Das Voranschreiten der Reaktion kann an der Bildung von Alkalimetallchlorid abgelesen oder dünnschichtchromatographisch verfolgt werden. Je nach Reaktivität des eingesetzten Thiolats ist die Umsetzung nach 3-12 h vollständig. Man gießt die Reaktionsmischung auf Wasser und extrahiert mit einem inerten organischen Lösungsmittel. Anschließend wird die organische Phase getrocknet und das Reinprodukt destillativ bzw. durch Filtration isoliert.

### Beispiel 1

In einem Zweihalskolben mit aufgesetztem Trockeneiskühler wurden 250 ml Dimethylformamid und 93,8 g einer 50 gew.-%igen wäßrigen Natriumhydroxidlösung gemischt und durch Einleiten von Argon der Sauerstoff weitgehend verdrängt. Anschließend setzte man 122 g Thiophenol zu und leitete bei einer Temperatur von ca. 60°C 130 g Chlortrifluorethan (R133a) in die Reaktionsmischung ein. Nach 16 h war die Umsetzung vollständig (DC-Kontrolle: Kieselgel, Dichlormethan). Man goß die Reaktionsmischung auf Wasser, extrahierte dreimal mit Dichlormethan und trocknete die vereinigten organischen Extrakte über Natriumsulfat. Anschließend wurde das Lösungsmittel abdestilliert, der ölige Rückstand im Vakuum destilliert (68°C/16 mbar), und man erhielt 134,5 g 2,2,2-Trifluorethylthiobenzol.

### Beispiel 2

In 60 ml trockenem Dimethylformamid wurden 11,1 g 2-Mercaptopyridin vorgelegt und langsam mit 4 g einer 60 gew.-%igen Suspension von Natriumhydrid in Paraffin versetzt. Anschließend kühlte man die Lösung auf 0°C und leitete 12 g Chlortrifluorethan ein. Man überführte die Reaktionsmischung in einen Rührautoklaven und erhitzte 12 h auf 100°C. Dabei stellte sich ein Maximaldruck von 2,5 bar ein. Das erkaltete Reaktionsgemisch wurde anschließend in Wasser gegossen und dreimal mit Dichlormethan extrahiert. Von der mit Na₂SO₄ getrockneten organischen Phase wurde das Lösungsmittel abdestilliert und der Rückstand im Vakuum fraktioniert (78°C/9,5 mbar). Man erhielt 8,0 g 2-(2',2',2'-Trifluorethylthio)pyridin als farbloses Öl.

### Beispiel 3

Man legte in einem Zweihalskolben 45 ml Dimethylformamid und 16 g einer 50 gew.-%igen wäßrigen Natriumhydroxidlösung unter Stickstoff vor. Nach Zugabe von 15 g 2-Mercaptobenzoesäure leitete man bei 0°C 18 g Chlortrifluorethan ein und überführte dann die Reaktionsmischung in einen Rührautoklaven. Man erhitzte 12 h auf 100°C (Maximaldruck 2,5 bar), ließ abkühlen und fällte dann das Rohprodukt durch Zugabe von konzentrierter Salzsäure. Das getrocknete Rohprodukt wurde aus heißer Ameisensäure umkristallisiert und man erhielt 5,3 g 2-(2',2',2'-Trifluorethylthio)benzoesäure mit Schmelzpunkt 226-227°C.

### Beispiel 4

In einem Zweihalskolben mit aufgesetztem Trockeneiskühler wurden 2 g einer 60 gew.-%igen Suspension von Natriumhydrid in Paraffin in 50 ml trockenem Dimethylformamid vorgelegt und anschließend 7,3 g 4-Chlorthiophenol in 10 ml Dimethylformamid zugetropft. Danach wurde Chlortrifluorethan bei einer Reaktionstemperatur von ca. 60°C eingeleitet, bis das Gas am Trockeneiskühler zu kondensierten begann. Man rührte weitere 5 h und verfolgte den Reaktionsverlauf dünnschichtchromatographisch (Kieselgel; Essigsäureethylester/Petrolether 1/5). Zur Aufarbeitung goß man die Reaktionsmischung in Wasser und extrahierte mit Dichlormethan. Die vereinigten organischen Extrakte wurden getrocknet und das Lösungmittel bei reduziertem Druck abdestilliert. Der ölige Rückstand wurde anschließend destilliert (43-45°C/0,1 mbar), und man erhielt 8,8 g 4-Chlor-(2',2',2'-trifluorethylthio)-benzol.

### Beispiel 5

In einem Zweilhalskolben mit aufgesetztem Trockeneiskühler wurden 65 ml Dimethylformamid mit 16 g einer 50 gew.-%igen Natriumhydroxidlösung unter Argon vorgelegt und anschließend 25 g 4-Aminothiophenol zugegeben. In die auf 60°C erwärmte klare Lösung leitete man anschließend 35 g Chlortrifluorethan ein. Man verfolgte den Reaktionsverlauf dünnschichtchromatographisch (Kieselgel; Dichlormethan) und goß nach ca. 3 h die Reaktionsmischung in Wasser. Es wurde mit Dichlormethan extrahiert, die vereinigten organischen Extrakte mit Na₂SO₄ getrocknet und das Reaktionsprodukt durch Einleiten von Chlorwasserstoff als Hydrochlorid gefällt. Man kristallisierte aus Essigsäureethylester um und erhielt 28 g reines 4-Amino-(2',2',2'-trifluorethylthio)benzol (HCl-Addukt) mit Schmelzpunkt 230-231°C (Zersetzung).

### Beispiel 6

In einer Apparatur wie in Beispiel 5 deprotonierte man 13 g p-Methylthiophenol mit 4 g Natriumhydrid (60 gew.-%igen Suspension in Paraffin) in 90 ml trockenem Dimethylformamid. Anschließend erwärmte man die Reaktionsmischung auf 60°C und leitete unter Rühren 23 g Chlortrifluorethan ein. Nach 5,5 h Rühren bei 50-60°C wurde die Reaktionsmischung auf Wasser gegossen und dreimal mit Dichlormethan extrahiert. Die organische Phase wurde zusätzlich mit Wasser und gesättigter Natriumhydrogencarbonatlösung gewaschen und dann über Natriumsulfat getrocknet. Das Lösungsmittel wurde bei reduziertem Druck abdestilliert und der Rückstand fraktioniert (49-51°C/1 mbar) Man erhielt 13,4 g 4-Methyl-(2',2',2'-trifluorethylthio)benzol als farbloses Öl.

### Beispiel 7

2 g Natriumhydrid wurden in 40 ml Dimethylformamid suspendiert, und bei 0°C wurden 8,4 g 2-Mercaptobenzthiazol in 20 ml Dimethylformamid zugetropft. Nach 30 min wurden bei der gleichen Temperatur 12 g R133a eingeleitet. Man überführte die Lösung in einen Rührautoklaven und erhitzte 18 h auf 100°C.

Dabei stellte sich ein Maximaldruck von 2,1 bar ein. Das erkaltete Reaktionsgemisch wurde anschließend in Wasser gegossen und dreimal mit Dichlormethan extrahiert. Von der mit Na₂SO₄ getrockneten organischen Phase wurde das Lösungsmittel abdestilliert und der Rückstand im Vakuum (80°C/0,05 mbar) fraktioniert. Man erhielt 5,1 g 2-(2',2',2'-Trifluorethylthio)benzthiazol.

### Beispiel 8

In einem Zweihalskolben wurden 150 ml Dimethylformamid und 40 g einer 50 gew.-%igen Natriumhydroxidlösung unter Stickstoff vorgelegt. Nach langsamer Zugabe von 62 g Benzylmercaptan ließ man die Reaktionslösung wieder auf 30°C abkühlen und leitete dann 80 g Chlortrifluorethan ein. Nach 0,5 h erhöhte man die Reaktionstemperatur auf 60°C und verfolgte den Reaktionsverlauf dünnschichtchromatographisch (Kieselgel; Petrolether/Dichlormethan 1/1). Nach 3 h ließ man den Ansatz auf Raumtemperatur abkühlen und goß dann den Kolbeninhalt in ca. 200 ml Wasser. Es wurde dreimal mit Dichlormethan extrahiert und die vereinigten organischen Extrakte dreimal mit Wasser und einmal mit gesättigter Natriumchloridlösung gewaschen. Nach Trocknung über Natriumsulfat wurde das Lösungsmittel bei reduziertem Druck abdestilliert und der Rückstand im Vakuum (57°C/1,5 mbar) destilliert. Man erhielt 92 g 2,2,2-Trifluorethylbenzylsulfid als farbloses Öl.

## Patentansprüche

1. Verfahren zur Herstellung von Trifluorethylschwefelverbindungen aus Thiolaten und 1-Chlor-2,2,2-trifluorethan, dadurch gekennzeichnet, daß man 1-Chlor-2,2,2-trifluorethan in einem polaren aprotischen Lösungsmittel auf Salze von Thiolen der Formel RSH einwirken läßt, wobei R ein unsubstituierter oder substituierter Rest mit folgender Bedeutung ist:
C₁-C₂₀-Alkyl
oder
C₃-C₁₂-Cycloalkyl
oder
ein einkerniger, kondensierter zweikerniger oder kondensierter dreikerniger Arylrest,
oder
ein gesättigter oder ungesättigter, ein- oder zweikerniger heterocyclischer Rest, der insgesamt ein oder zwei Heteroatome enthält, die unabhängig voneinander N, S oder O sein können.

2. Verfahren nach Anspruch 1, dadurch gekennzeichnet, daß R folgende Bedeutung hat:
C₁-C₂₀-Alkyl,
unsubstituiert oder substituiert mit einem oder zwei Resten aus der Gruppe Amino, F, C₁-C₃-Alkylamino, C₁-C₃-Dialkylamino, Hydroxyl, Carboxyl, C₁-C₆-Alkoxy, C₃-C₁₂-Cycloalkyl, Pyridyl, Imidazolyl, Thiazolyl, Oxazolyl, Phenyl, wobei Phenyl unsubstituiert oder mit einem oder zwei Resten aus der Gruppe F, Cl, Br, Amino, C₁-C₃-Alkylamino, C₁-C₃-Dialkylamino, Hydroxyl, Carboxyl, C₁-C₃-Alkoxy, C₁-C₆-Alkyl substituiert ist;
oder
C₃-C₁₂-Cycloalkyl,
unsubstituiert oder substituiert mit einem oder zwei Resten aus der Gruppe C₁-C₆-Alkyl, F, Amino, C₁-C₃-Alkylamino, C₁-C₃-Dialkylamino, Hydroxyl, Carboxyl, C₁-C₆-Alkoxy, Pyridyl, Imidazolyl, Thiazolyl, Oxazolyl, Phenyl, wobei Phenyl unsubstituiert oder mit einem oder zwei Resten aus der Gruppe F, Cl, Br, Amino, C₁-C₃-Alkylamino, C₁-C₃-Dialkylamino, Hydroxyl, Carboxyl, C₁-C₃-Alkoxy, C₁-C₆-Alkyl substituiert ist;
oder
ein einkerniger, kondensierter zweikerniger oder kondensierter dreikerniger Arylrest,
unsubstituiert oder substituiert mit einem oder zwei Resten aus der Gruppe C₁-C₆-Alkyl, F, Cl, Br, Amino, C₁-C₃-Alkylamino, C₁-C₃-Dialkylamino, Hydroxyl, Carboxyl, C₁-C₆-Alkoxy;
oder
ein gesättigter oder ungesättigter, ein- oder zweikerniger heterocyclischer Rest, der insgesamt ein oder zwei Heteroatome enthält, die unabhängig voneinander N, S oder O sein können,
wobei der Rest unsubstituiert oder substituiert ist mit einem oder zwei Resten aus der Gruppe C₁-C₆-Alkyl, F, Amino, C₁-C₃-Alkylamino, C₁-C₃-Dialkylamino, Hydroxyl, Carboxyl, C₁-C₆-Alkoxy, sowie, falls der heterocyclische Rest aromatisch ist, Cl und Br.

3. Verfahren nach Anspruch 1, dadurch gekennzeichnet, daß R folgende Bedeutung hat:
C₁-C₁₂-Alkyl,
unsubstituiert oder substituiert mit einem oder zwei Resten aus der Gruppe F, Amino, C₁-C₃-Alkylamino, C₁-C₃-Dialkylamino, Hydroxyl, Carboxyl, C₁-C₃-Alkoxy, C₃-C₈-Cycloalkyl, Pyridyl, Imidazolyl, Thiazolyl, Oxazolyl, Phenyl, wobei Phenyl unsubstituiert oder mit einem oder zwei Resten aus der Gruppe F, Cl, Br, Amino, C₁-C₃-Alkylamino, C₁-C₃-Dialkylamino, Hydroxyl, Carboxyl, C₁-C₃-Alkoxy, C₁-C₄-Alkyl substituiert ist;
oder
C₃-C₈-Cycloalkyl,
unsubstituiert oder substituiert mit einem oder zwei Resten aus der Gruppe C₁-C₄-Alkyl, F, Amino, C₁-C₃-Alkylamino, C₁-C₃-Dialkylamino, Hydroxyl, Carboxyl, C₁-C₃-Alkoxy, Pyridyl, Imidazolyl, Thiazolyl, Oxazolyl, Phenyl, wobei Phenyl unsubstituiert oder mit einem oder zwei Resten aus der Gruppe F, Cl, Br, Amino, C₁-C₃-Alkylamino, C₁-C₃-Dialkylamino, Hydroxyl, Carboxyl, C₁-C₃-Alkoxy, C₁-C₄-Alkyl substituiert ist;
oder
ein einkerniger, kondensierter zweikerniger oder kondensierter dreikerniger Arylrest,
unsubstituiert oder substituiert mit einem oder zwei Resten aus der Gruppe C₁-C₄-Alkyl, F, Cl, Br, Amino, C₁-C₃-Alkylamino, C₁-C₃-Dialkylamino, Hydroxyl, Carboxyl, C₁-C₃-Alkoxy;
oder
ein gesättigter oder ungesättigter, ein- oder zweikerniger heterocyclischer Rest, der insgesamt ein oder zwei Heteroatome enthält, die unabhängig voneinander N, S oder O sein können,
wobei der Rest unsubstituiert oder substituiert ist mit einem oder zwei Resten aus der Gruppe C₁-C₄-Alkyl, F, Amino, C₁-C₃-Alkylamino, C₁-C₃-Dialkylamino, Hydroxyl, Carboxyl, C₁-C₃-Alkoxy, sowie, falls der heterocyclische Rest aromatisch ist, Cl und Br.

4. Verfahren nach Anspruch 1, dadurch gekennzeichnet, daß R folgende Bedeutung hat:
C₁-C₆-Alkyl,
unsubstituiert oder substituiert mit einem oder zwei Resten aus der Gruppe F, Amino, C₁-C₃-Alkylamino, C₁-C₃-Dialkylamino, Hydroxyl, Carboxyl, C₁-C₃-Alkoxy, C₃-C₈-Cycloalkyl, Pyridyl, Imidazolyl, Thiazolyl, Oxazolyl, Phenyl, wobei Phenyl unsubstituiert oder mit einem oder zwei Resten aus der Gruppe F, Cl, Br, C₁-C₃-Alkyl, C₁-C₃-Alkoxy substituiert ist;
oder
C₃-C₆-Cycloalkyl,
unsubstituiert oder substituiert mit einem oder zwei Resten aus der Gruppe C₁-C₄-Alkyl, F, Amino, C₁-C₃-Alkylamino₁ C₁-C₃-Dialkylamino, Hydroxyl, Carboxyl, C₁-C₃-Alkoxy, Pyridyl, Imidazolyl, Thiazolyl, Oxazolyl, Phenyl;
oder
Phenyl,
unsubstituiert oder substituiert mit einem oder zwei Resten aus der Gruppe C₁-C₃-Alkyl, F, Cl, Br, Amino, C₁-C₃-Alkylamino, C₁-C₃-Dialkylamino, Hydroxyl, Carboxyl, C₁-C₃-Alkoxy;
oder
ein ungesättigter ein- oder zweikerniger heterocyclischer Rest, der insgesamt ein oder zwei Heteroatome enthält, die unabhängig voneinander N, S oder O sein können,
wobei der Rest unsubstituiert oder substituiert ist mit einem oder zwei Resten aus der Gruppe C₁-C₃-Alkyl, F, Amino, C₁-C₃-Alkylamino, C₁-C₃-Dialkylamino, Hydroxyl, Carboxyl, C₁-C₃-Alkoxy, sowie, falls der heterocyclische Rest aromatisch ist, Cl und Br.

5. Verfahren nach Anspruch 1, dadurch gekennzeichnet, daß R folgende Bedeutung hat:
C₁-C₄-Alkyl;
oder
C₃-C₆-Cycloalkyl;
oder
Phenyl,
unsubstituiert oder substituiert mit einem oder zwei Resten aus der Gruppe Methyl, F, Cl, Br, Amino, C₁-C₃-Alkylamino, C₁-C₃-Dialkylamino, Hydroxyl, Carboxyl, Methoxy;
oder
Pyridyl, Imidazolyl, Benzimidazolyl, Thiazolyl, Benzthiazolyl, Oxazolyl oder Benzoxazolyl.

6. Verfahren nach einem der Ansprüche 1 bis 5, dadurch gekennzeichnet, daß man als polares aprotisches Lösungsmittel N-Methylpyrrolidon, N-Methylformamid, Dimethylformamid, Dimethylacetamid, Sulfolan, Dimethylsulfoxid, Butyrolacton oder Tetrahydrofuran einsetzt.

7. Verfahren nach einem der Ansprüche 1 bis 6, dadurch gekennzeichnet, daß man bei Atmosphärendruck oder einem Druck bis 5 bar arbeitet.

## Claims

1. A process for preparing trifluoroethyl sulfur compounds from thiolates and 1-chloro-2,2,2-trifluoroethane, which comprises allowing 1-chloro-2,2,2-trifluoroethane to act in a polar aprotic solvent upon salts of thiols of the formula RSH, where R is an unsubstituted or substituted radical as defined below:
C₁-C₂₀-alkyl
or
C₃-C₁₂-cycloalkyl
or
a monocyclic, condensed dicyclic or condensed tricyclic aryl radical,
or
a saturated or unsaturated monocyclic or dicyclic heterocyclic radical, which contains a total of one or two heteroatoms, which can, independently of one another, be N, S or O.

2. The process as claimed in claim 1, wherein R is as defined below:
C₁-C₂₀-alkyl,
unsubstituted or substituted by one or two radicals selected from the group consisting of amino, F, C₁-C₃-alkylamino, C₁-C₃-dialkylamino, hydroxyl, carboxyl, C₁-C₆-alkoxy, C₃-C₁₂-cycloalkyl, pyridyl, imidazolyl, thiazolyl, oxazolyl, phenyl, with phenyl being unsubstituted or substituted by one or two radicals selected from the group consisting of F, Cl, Br, amino, C₁-C₃-alkylamino, C₁-C₃-dialkylamino, hydroxyl, carboxyl, C₁-C₃-alkoxy, C₁-C₆-alkyl;
or
C₃-C₁₂-cycloalkyl,
unsubstituted or substituted by one or two radicals selected from the group consisting of C₁-C₆-alkyl, F, amino, C₁-C₃-alkylamino, C₁-C₃-dialkylamino, hydroxyl, carboxyl, C₁-C₆-alkoxy, pyridyl, imidazolyl, thiazolyl, oxazolyl, phenyl, with phenyl being unsubstituted or substituted by one or two radicals selected from the group consisting of F, Cl, Br, amino, C₁-C₃-alkylamino, C₁-C₃-dialkylamino, hydroxyl, carboxyl, C₁-C₃-alkoxy, C₁-C₆-alkyl;
or
a monocyclic, condensed dicyclic or condensed tricyclic aryl radical,
unsubstituted or substituted by one or two radicals selected from the group consisting of C₁-C₆-alkyl, F, Cl, Br, amino, C₁-C₃-alkylamino, C₁-C₃-dialkylamino, hydroxyl, carboxyl, C₁-C₆-alkoxy;
or
a saturated or unsaturated, monocyclic or dicyclic heterocyclic radical, which contains a total of one or two heteroatoms, which can, independently of one another, be N, S or O,
where the radical is unsubstituted or substituted by one or two radicals selected from the group consisting of C₁-C₆-alkyl, F, amino, C₁-C₃-alkylamino, C₁-C₃-dialkylamino, hydroxyl, carboxyl, C₁-C₆-alkoxy, and also, if the heterocyclic radical is aromatic, Cl and Br.

3. The process as claimed in claim 1, wherein R is as defined below:
C₁-C₁₂-alkyl,
unsubstituted or substituted by one or two radicals selected from the group consisting of F, amino, C₁-C₃-alkylamino, C₁-C₃-dialkylamino, hydroxyl, carboxyl, C₁-C₃-alkoxy, C₃-C₈-cycloalkyl, pyridyl, imidazolyl, thiazolyl, oxazolyl, phenyl, with phenyl being unsubstituted or substituted by one or two radicals selected from the group consisting of F, Cl, Br, amino, C₁-C₃-alkylamino, C₁-C₃-dialkylamino, hydroxyl, carboxyl, C₁-C₃-alkoxy, C₁-C₄-alkyl;
or
C₃-C₈-cycloalkyl,
unsubstituted or substituted by one or two radicals selected from the group consisting of C₁-C₄-alkyl, F, amino, C₁-C₃-alkylamino, C₁-C₃-dialkylamino, hydroxyl, carboxyl, C₁-C₃-alkoxy, pyridyl, imidazolyl, thiazolyl, oxazolyl, phenyl, with phenyl being unsubstituted or substituted by one or two radicals selected from the group consisting of F, Cl, Br, amino, C₁-C₃-alkylamino, C₁-C₃-dialkylamino, hydroxyl, carboxyl, C₁-C₃-alkoxy, C₁-C₄-alkyl;
or
a monocyclic, condensed dicyclic or condensed tricyclic aryl radical,
unsubstituted or substituted by one or two radicals selected from the group consisting of C₁-C₄-alkyl, F, Cl, Br, amino, C₁-C₃-alkylamino, C₁-C₃-dialkylamino, hydroxyl, carboxyl, C₁-C₃-alkoxy;
or
a saturated or unsaturated, monocyclic or dicyclic heterocyclic radical, which contains a total of one or two heteroatoms, which can, independently of one another, be N, S or O,
where the radical is unsubstituted or substituted by one or two radicals selected from the group consisting of C₁-C₄-alkyl, F, amino, C₁-C₃-alkylamino, C₁-C₃-dialkylamino, hydroxyl, carboxyl, C₁-C₃-alkoxy, and also, if the heterocyclic radical is aromatic, Cl and Br.

4. The process as claimed in claim 1, wherein R is as defined below:
C₁-C₆-alkyl,
unsubstituted or substituted by one or two radicals selected from the group consisting of F, amino, C₁-C₃-alkylamino, C₁-C₃-dialkylamino, hydroxyl, carboxyl, C₁-C₃-alkoxy, C₃-C₈-cycloalkyl, pyridyl, imidazolyl, thiazolyl, oxazolyl, phenyl, with phenyl being unsubstituted or substituted by one or two radicals selected from the group consisting of F, Cl, Br, C₁-C₃-alkyl, C₁-C₃-alkoxy;
or
C₃-C₆-cycloalkyl,
unsubstituted or substituted by one or two radicals selected from the group consisting of C₁-C₄-alkyl, F, amino, C₁-C₃-alkylamino, C₁-C₃-dialkylamino, hydroxyl, carboxyl, C₁-C₃-alkoxy, pyridyl, imidazolyl, thiazolyl, oxazolyl, phenyl;
or
phenyl,
unsubstituted or substituted by one or two radicals selected from the group consisting of C₁-C₃-alkyl, F, Cl, Br, amino, C₁-C₃-alkylamino, C₁-C₃-dialkylamino, hydroxyl, carboxyl, C₁-C₃-alkoxy;
or
an unsaturated monocyclic or dicyclic heterocyclic radical, which contains a total of one or two heteroatoms, which can, independently of one another, be N, S or O,
where the radical is unsubstituted or substituted by one or two radicals selected from the group consisting of C₁-C₃-alkyl, F, amino, C₁-C₃-alkylamino, C₁-C₃-dialkylamino, hydroxyl, carboxyl, C₁-C₃-alkoxy, and also, if the heterocyclic radical is aromatic, Cl and Br.

5. The process as claimed in claim 1, wherein R is as defined below:
C₁-C₄-alkyl;
or
C₃-C₆-cycloalkyl;
or
phenyl,
unsubstituted or substituted by one or two radicals selected from the group consisting of methyl, F, Cl, Br, amino, C₁-C₃-alkylamino, C₁-C₃-dialkylamino, hydroxyl, carboxyl, methoxy;
or
pyridyl, imidazolyl, benzimidazolyl, thiazolyl, benzothiazolyl, oxazolyl or benzoxazolyl.

6. The process as claimed in any one of claims 1 to 5, wherein the polar aprotic solvent used is N-methylpyrrolidone, N-methylformamide, dimethylformamide, dimethylacetamide, sulfolane, dimethyl sulfoxide, butyrolactone or tetrahydrofuran.

7. The process as claimed in any one of claims 1 to 6, wherein the process is carried out at atmospheric pressure or under a pressure of up to 5 bar.

## Revendications

1. Procédé pour la préparation de composés de trifluoroéthyl-soufre à partir de thiolates et du 1-chloro-2,2,2-trifluoroéthane, caractérisé en ce qu'on laisse agir du 1-chloro-2,2,2-trifluoroéthane dans un solvant aprotique polaire sur des sels de thiols répondant à la formule RSH dans laquelle R représente un radical substitué ou non avec la signification suivante :
un groupe alkyle en C₁ -C₂₀
ou
un groupe cycloalkyle en C₃-C₁₂
ou
un radical aryle mononucléaire, binucléaire condensé ou trinucléaire condensé,
ou
un radical hétérocyclique mono- ou binucléaire, saturé ou insaturé, qui contient au total un ou deux hétéroatomes qui peuvent représenter, indépendamment l'un de l'autre, N, S ou O.

2. Procédé selon la revendication 1, caractérisé en ce que R représente les groupes indiqués ci-après :
un groupe alkyle en C₁ -C₂₀,
non substitué ou portant à titre de substituants un ou deux radicaux choisis parmi le groupe comprenant un groupe amino, un atome de fluor, un groupe alkyl(en C₁-C₃)amino, un groupe dialkyl(en C₁-C₃)amino, un groupe hydroxyle, un groupe carboxyle, un groupe alcoxy en C₁-C₆, un groupe cycloalkyle en C₃-C₁₂, un groupe pyridyle, un groupe imidazolyle, un groupe thiazolyle, un groupe oxazolyle, un groupe phényle, le groupe phényle étant non substitué ou portant à titre de substituants un ou deux radicaux choisis parmi le groupe comprenant un atome de fluor, un atome de chlore, un atome de brome, un groupe amino, un groupe alkyl(en C₁-C₃)amino, un groupe dialkyl(en C₁-C₃) amino, un groupe hydroxyle, un groupe carboxyle, un groupe alcoxy en C₁-C₃, un groupe alkyle en C₁-C₆;
ou
un groupe cycloalkyle en C₃-C₁₂
non substitué ou portant à titre de substituants un ou deux radicaux choisis parmi le groupe comprenant un groupe alkyle en C₁-C₆, un atome de fluor, un groupe amino, un groupe alkyl(en C₁-C₃)amino, un groupe dialkyl(en C₁-C₃)amino, un groupe hydroxyle, un groupe carboxyle, un groupe alcoxy en C₁-C₆, un groupe pyridyle, un groupe imidazolyle, un groupe thiazolyle, un groupe oxazolyle, un groupe phényle, le groupe phényle étant non substitué ou portant à titre de substituants un ou deux radicaux choisis parmi le groupe comprenant un atome de fluor, un atome de chlore, un atome de brome, un groupe amino, un groupe alkyl(en C₁-C₃)amino, un groupe dialkyl(en C₁-C₃) amino, un groupe hydroxyle, un groupe carboxyle, un groupe alcoxy en C₁-C₃, un groupe alkyle en C₁-C₆;
ou
un radical aryle mononucléaire, binucléaire condensé ou trinucléaire condensé,
non substitué ou portant à titre de substituants un ou deux radicaux choisis parmi le groupe comprenant un groupe alkyle en C₁-C₆, un atome de fluor, un atome de chlore, un atome de brome, un groupe amino, un groupe alkyl(en C₁-C₃)amino, un groupe dialkyl(en C₁-C₃) amino, un groupe hydroxyle, un groupe carboxyle, un groupe alcoxy en C₁-C₆;
ou
un radical hétérocyclique mono- ou binucléaire, saturé ou insaturé, qui contient au total un ou deux hétéroatomes qui peuvent représenter, indépendamment l'un de l'autre, N, S ou O,
le radical étant non substitué ou portant à titre de substituants un ou deux radicaux choisis parmi le groupe comprenant un groupe alkyle en C₁-C₆, un atome de fluor, un groupe amino, un groupe alkyl(en C₁-C₃) amino, un groupe dialkyl(en C₁-C₃)amino, un groupe hydroxyle, un groupe carboxyle, un groupe alcoxy en C₁-C₆, ainsi que, au cas où le radical hétérocyclique est aromatique, un atome de chlore et un atome de brome.

3. Procédé selon la revendication 1, caractérisé en ce que R représente les groupes indiqués ci-après :
un groupe alkyle en C₁-C₁₂,
non substitué ou portant à titre de substituants un ou deux radicaux choisis parmi le groupe comprenant un atome de fluor, un groupe amino, un groupe alkyl(en C₁-C₃)amino, un groupe dialkyl(en C₁-C₃)amino, un groupe hydroxyle, un groupe carboxyle, un groupe alcoxy en C₁-C₃, un groupe cycloalkyle en C₃-C₈, un groupe pyridyle, un groupe imidazolyle, un groupe thiazolyle, un groupe oxazolyle, un groupe phényle, le groupe phényle étant non substitué ou portant à titre de substituants un ou deux radicaux choisis parmi le groupe comprenant un atome de fluor, un atome de chlore, un atome de brome, un groupe amino, un groupe alkyl(en C₁-C₃)amino, un groupe dialkyl(en C₁-C₃)amino, un groupe hydroxyle, un groupe carboxyle, un groupe alcoxy en C₁-C₃, un groupe alkyle en C₁-C₄;
ou
un groupe cycloalkyle en C₃-C₈
non substitué ou portant à titre de substituants un ou deux radicaux choisis parmi le groupe comprenant un groupe alkyle en C₁-C₄, un atome de fluor, un groupe amino, un groupe alkyl(en C₁-C₃)amino, un groupe dialkyl(en C₁-C₃)amino, un groupe hydroxyle, un groupe carboxyle, un groupe alcoxy en C₁-C₃, un groupe pyridyle, un groupe imidazolyle, un groupe thiazolyle, un groupe oxazolyle, un groupe phényle, le groupe phényle étant non substitué ou portant à titre de substituants un ou deux radicaux choisis parmi le groupe comprenant un atome de fluor, un atome de chlore, un atome de brome, un groupe amino, un groupe alkyl(en C₁-C₃)amino, un groupe dialkyl(en C₁-C₃) amino, un groupe hydroxyle, un groupe carboxyle, un groupe alcoxy en C₁-C₃, un groupe alkyle en C₁-C₄;
ou
un radical aryle mononucléaire, binucléaire condensé ou trinucléaire condensé,
non substitué ou portant à titre de substituants un ou deux radicaux choisis parmi le groupe comprenant un groupe alkyle en C₁-C₄, un atome de fluor, un atome de chlore, un atome de brome, un groupe amino, un groupe alkyl(en C₁-C₃)amino, un groupe dialkyl(en C₁-C₃) amino, un groupe hydroxyle, un groupe carboxyle, un groupe alcoxy en C₁-C₃;
ou
un radical hétérocyclique mono- ou binucléaire, saturé ou insaturé, qui contient au total un ou deux hétéroatomes qui peuvent représenter, indépendamment l'un de l'autre, N, S ou O,
le radical étant non substitué ou portant à titre de substituants un ou deux radicaux choisis parmi le groupe comprenant un groupe alkyle en C₁-C₄, un atome de fluor, un groupe amino, un groupe alkyl(en C₁-C₃) amino, un groupe dialkyl(en C₁-C₃)amino, un groupe hydroxyle, un groupe carboxyle, un groupe alcoxy en C₁-C₃, ainsi que, au cas où le radical hétérocyclique est aromatique, un atome de chlore et un atome de brome.

4. Procédé selon la revendication 1, caractérisé en ce que R représente les groupes indiqués ci-après :
un groupe alkyle en C₁-C₆,
non substitué ou portant à titre de substituants un ou deux radicaux choisis parmi le groupe comprenant un atome de fluor, un groupe amino, un groupe alkyl(en C₁-C₃)amino, un groupe dialkyl(en C₁-C₃)amino, un groupe hydroxyle, un groupe carboxyle, un groupe alcoxy en C₁-C₃, un groupe cycloalkyle en C₃-C₈, un groupe pyridyle, un groupe imidazolyle, un groupe thiazolyle, un groupe oxazolyle, un groupe phényle, le groupe phényle étant non substitué ou portant à titre de substituants un ou deux radicaux choisis parmi le groupe comprenant un atome de fluor, un atome de chlore, un atome de brome, un groupe alkyle en C₁-C₃, un groupe alcoxy en C₁-C₃;
ou
un groupe cycloalkyle en C₃-C₆
non substitué ou portant à titre de substituants un ou deux radicaux choisis parmi le groupe comprenant un groupe alkyle en C₁-C₄, un atome de fluor, un groupe amino, un groupe alkyl(en C₁-C₃)amino, un groupe dialkyl(en C₁-C₃)amino, un groupe hydroxyle, un groupe carboxyle, un groupe alcoxy en C₁-C₃, un groupe pyridyle, un groupe imidazolyle, un groupe thiazolyle, un groupe oxazolyle, un groupe phényle;
ou
un groupe phényle
non substitué ou portant à titre de substituants un ou deux radicaux choisis parmi le groupe comprenant un groupe alkyle en C₁-C₃, un atome de fluor, un atome de chlore, un atome de brome, un groupe amino, un groupe alkyl(en C₁-C₃)amino, un groupe dialkyl(en C₁-C₃) amino, un groupe hydroxyle, un groupe carboxyle, un groupe alcoxy en C₁-C₃,
ou
un radical hétérocyclique mono- ou binucléaire insaturé qui contient au total un ou deux hétéroatomes qui peuvent représenter, indépendamment l'un de l'autre, N, S ou O,
le radical étant non substitué ou portant à titre de substituants un ou deux radicaux choisis parmi le groupe comprenant un groupe alkyle en C₁-C₃, un atome de fluor, un groupe amino, un groupe alkyl(en C₁-C₃) amino, un groupe dialkyl(en C₁-C₃)amino, un groupe hydroxyle, un groupe carboxyle, un groupe alcoxy en C₁-C₃, ainsi que, au cas où le radical hétérocyclique est aromatique, un atome de chlore et un atome de brome.

5. Procédé selon la revendication 1, caractérisé en ce que R représente les groupes indiqués ci-après :
un groupe alkyle en C₁-C₄,
ou
un groupe cycloalkyle en C₃-C₆
ou
un groupe phényle
non substitué ou portant à titre de substituants un ou deux radicaux choisis parmi le groupe comprenant un groupe méthyle, un atome de fluor, un atome de chlore, un atome de brome, un groupe amino, un groupe alkyl(en C₁-C₃)amino, un groupe dialkyl(en C₁-C₃)amino, un groupe hydroxyle, un groupe carboxyle, un groupe méthoxy,
ou
un groupe pyridyle, un groupe imidazolyle, un groupe benzimidazolyle, un groupe thiazolyle, un groupe benzthiazolyle, un groupe oxazolyle ou un groupe benzoxazolyle.

6. Procédé selon l'une quelconque des revendications 1 à 5, caractérisé en ce qu'on met en oeuvre à titre de solvant aprotique polaire la N-méthylpyrrolidone, le N-méthylformamide, le diméthylformamide, le diméthylacétamide, le sulfolane, le diméthylsulfoxyde, la butyrolactone ou le tétrahydrofuranne.

7. Procédé selon l'une quelconque des revendications 1 à 6, caractérisé en ce qu'on travaille sous la pression atmosphérique ou sous une pression allant jusque 5 bar.
